Europäisches Patentamt

European Patent Office

Office européen des brevets

(11) Publication number: **0 357 830**
**A1**

## EUROPEAN PATENT APPLICATION

(21) Application number: 88114788.8

(22) Date of filing: 09.09.88

(51) Int. Cl.5: **B01J 29/34** , **C07C 1/04**

(43) Date of publication of application:
14.03.90 Bulletin 90/11

(84) Designated Contracting States:
**BE CH DE FR GB LI NL**

(71) Applicant: **UNION CARBIDE CORPORATION**
39 Old Ridgebury Road
Danbury Connecticut 06817(US)

(72) Inventor: **Miller, James George**
95 South Magnolia Avenue
Pearl River New York 10965(US)
Inventor: **Rabo, Jule Anthony**
19 Windmill Road
Armonk New York 10504(US)

(74) Representative: **Eggert, Hans-Gunther, Dr.**
Räderscheidtstrasse 1
D-5000 Köln 41(DE)

(54) Cobalt fischer-tropsch catalysts having improved selectivity.

(57) A cobalt Fischer-Tropsch catalyst having an improved steam treated, acid extracted LZ-210 support is taught. The new catalyst system demonstrates improved product selectivity at Fischer-Tropsch reaction conditions evidenced by lower methane production, higher $C_5^+$ yield and increased olefin production.

EP 0 357 830 A1

# COBALT FISCHER-TROPSCH CATALYSTS HAVING IMPROVED SELECTIVITY

## STATEMENT

The Government of the United States of America has rights to this invention pursuant to Contract No. DE-AC22-84PC70028 awarded by the U.S. Department of Energy.

## Field of the Invention

The present application relates to the field of cobalt Fischer-Tropsch catalysts in combination with an improved molecular sieve support.

## Background of the Invention

Iron Fischer-Tropsch (F-T) catalysts have generally been preferred commercially over cobalt based catalysts and are presently the only commerical F-T catalyst used.

In the conversion of syngas (CO + $H_2$ mixture) in the Fischer-Tropsch reaction cobalt catalysts have the benefit of higher activity and better selectivity to motor fuels, they suffer from their inherent production of excess methane (an undesirable product) as well as the paraffinic nature of the product. It would be an important catalyst improvement if a stable cobalt F-T catalyst was discovered which demonstrated reduced methane production, increased C5+ yield and improved olefin content, especially in the C5-range.

There is a reasonable amount of prior art dealing with Fischer-Tropsch metals combined with molecular sieve components. The most recent prior art pertinent to this invention is a patent issued to J.A. Rabo et al., U.S. Patent No. 4,652,538, and the prior art cited within.

An excellent review of past publications on cobalt Fischer-Tropsch catalyst was reported by R.B. Anderson, "The Fischer-Tropsch Synthesis", Academic Press, Orlando FL, 1984. In the review are lists of promoters and catalysts studied in the past, included are sightings of the use of Mn and Zr promoters. Listed below is a summary of the information presented in this article related to the use of the Mn and Zr promoters. Fischer and Koch showed Mn added to a cobalt kieselguhr catalyst was effective at shifting the product distribution toward heavier product as was also observed for the catalysts in this invention. Work by Eidus and Bulanova showed a similar effect on the addition of $ZrO_2$ to the same type of catalyst, this was not observed upon our addition of $ZrO_2$ to the Mn promoted Co/TC-123 catalyst system. No work to our knowledge has been reported on the use of a combined Mn and Zr promoted catalyst.

Dent, A.L. and Lin, M., Adv. Chem. Ser. 178,47 (1979) reported that the addition of Mn to a cobalt alumina catalyst increased the olefin content in the product, which is consistent with our data.

The only prior art of which the applicants are aware relating to Zr for improving the stability of a cobalt F-T catalyst was reported by Eidus and Bulanova U.S.S.R. 150,102, Sept. 26, 1962, Appl. Nov. 27, 1954. In this work Zr was used in place of thorium to reduce the sensitivity of the catalyst to super heating.

## Summary of the Invention

The present invention is directed to a cobalt Fischer-Tropsch Catalyst/Molecular Sieve combination which incorporates a newly developed molecular sieve which demonstrates improved performance as a catalyst support over previously known molecular sieve supports.

It has been found that the resultant catalyst demonstrates superior product selectivity and high stability which is evidenced by lower methane production, higher C5+ yields, increased olefin production, and longer catalyst life.

## Description of the Invention

We have found a new zeolitic molecular sieve based support which greatly reduces the undesirable methane production of a cobalt Fischer-Tropsch catalyst combined with it. This decrease in methane production results from shifting of the product slate toward heavier more desirable product. A significant increase in product produced above the motor fuels range is observed, however, this is not considered a draw back since these heavier products could easily be hydroprocessed back into the motor fuel boiling point range. The molecular sieve support acts to reduce the hydrogenation ability of the cobalt catalyst, promoting hydrocarbon chain growth over chain termination. Consistent with this theory the olefin content of the F-T product was increased, improving the value of the product, especially in the C5-range.

The molecular sieve support pertaining to this invention, denoted TC-123 consists of a steam treated, acid extracted form of the molecular sieve known as LZ-210 disclosed in U.S. Patent Nos. 4,503,023 and 4,610,856. The best way known to practice this invention is to start with an LZ-210 with a SiO2/Al2O3 ratio of 8.0 or above. The preferred procedure for producing the LZ-210 material used in the present invention is disclosed by Staniulis in commonly assigned co-pending application Serial No.      , filed concurrently herewith. This procedure involves subjecting a Y-zeolite to an LZ-210 secondary synthesis procedure without a subsequent ammonium exchange step. This material may then be ammonium exchanged and is steamed at $750^\circ C$ for 1hr in 100% steam followed by acid extraction in 3M HCL solution for 3hrs under reflux conditions. The resultant material has the following properties: $SiO2/Al2O3 = 200$, surface area (1 pt. BET0 = 800m2/g, and a x-ray A zero = 24.260.

The support is subsequently pore filled with a solution such as ethylene glycol, water or alcohol containing $Co(NO3)2$ and a $Mn(NO3)4$ promoter to give a theoretical percentage of $Co = 8.2\%$ and $Mn = 1.6\%$. The method of silica binding and shaping is not thought to be particularly important, in the context of the improvement taught in the present invention.

Characterization of the catalyst shows the promoted cobalt oxide encapsulated within the secondary pore structure of the TC-123 molecular sieve which was generated by the steam and acid extraction treatments. The secondary pores aid in tailoring and maintaining the cobalt particles at the optimum size for maximum activity and selectivity while allowing the syngas and products to diffuse through the primary zeolite pore structure.

The use of the novel catalyst system comprising cobalt and a steam treated acid extracted LZ-210 support as a Fischer-Tropsch catalyst is also taught.

The catalyst system of the present invention can most effectively be employed when promoted with a suitable Fischer-Tropsch catalyst promoter such as for example, Mn oxide, Zr Oxide and combinations thereof.

## EXAMPLES

While the invention has been described above, the details of the present invention will be better understood by recourse of the following examples which serve to illustrate that for a Mn promted cobalt F-T catalyst the use of a steam treated acid extracted LZ-210 molecular sieve support yields enhanced results when compared to the same catalyst over a known acid extracted UHP-Y molecular sieve (U.S. Patent No. 4,401,556 Bezman et al., and U.S. Patent No. 4,652,538 Rabo et al).

The following examples compare a manganese promted acid extracted UHP-Y supported F-T catalyst with the same catalyst using a steam treated, acid extracted LZ-210 molecular sieve support.

The catalysts were both prepared by the same procedure and run under identical conditions.

## EXAMPLE I

The catalyst was prepared by pore filling 100.0g anhydrous TC-123 with a warm ethylene glycol solution of 7.21g Mn(NO3)2 xH2O, 55.8g Co(NO3)2 6H2O, and 48.0g ethylene glycol. The catalyst was dried and calcined in air by the following procedure: 100C for 10hrs, ramp to 200C in 0.5hr, soak at 200C for 0.5hr, ramp to 450C in 1.25hrs, soak at 450C for 4hrs. The resultant powder was silica bonded and extruded into 1/8" extrudates, dried at 110C overnight followed calcination at 250C for 2hrs. The calculated percent components in the catalyst based on raw materials used were: Co = 8.2, Mn = 1.6, silica

3

binder = 15%, and TC-123 = 72.1.

A 80cc sample was loaded into a Berty reactor were it was treated with H2 at 300psig at 350C for 18hrs and exposed to 1:1 H2:CO syngas at 220C. The catalyst was tested at 240C and 260C under various pressures and H2:CO ratios. The results of the tests are contained in Table 1.


EXAMPLE II


A second catalyst was prepared using the same method as that described in Example I, substituting acid extracted UHP-Y molecular sieve for the TC-123 molecular sieve of Example I. The catalyst sample was tested in the same manner as in Example I, with the results shown in Table 1.

Table 1

| Comparison of Co/Mn F-T Catalysts | | |
|---|---|---|
| Example # | I | II |
| Catalyst Support | TC-123 | Acid Extracted UHP-Y |
| Conversion (H2 + CO) | 47.8 | 42.8 |
| CH4 | 3.3 | 5.3 |
| C2-C4 | 7.8 | 9.7 |
| C5-350F | 21.4 | 23.0 |
| 350-650F | 29.0 | 31.5 |
| 650F + | 38.6 | 30.4 |
| C5 + | 88.9 | 85.0 |
| C4 olefin/parifin | 3.7 | 2.1 |
| Conditions: 240C, 1:1 H2:CO, 300psig, 300GHSV | | |

The TC-123 supported catalyst demonstrated a substantial reduction in methane and C2-C4 production compared to the UHP-Y supported catalyst while demonstrating comparable or higher syngas conversion. The reduced C1-C4 fraction was realized in the more valuable C5 + fraction. The olefin content of the product was also found to be higher than the UHP-Y case as indicated by the olefin to paraffin ratio of the C4 fraction.

While the invention has been described with respect to various specific examples and embodiments, it is to be understood that the invention is not limited thereto and that it can be variously practiced within the scope of the following claims.

**Claims**

1. A cobalt Fischer-Tropsch catalyst supported by a steam treated, acid extracted LZ-210 molecular sieve.

2. A catalyst according to claim 1 wherein the acid extracted LZ-210 has a $SiO_2/Al_2O_3$ ratio of 8.0 or above.

3. A catalyst according to claim 1 wherein the LZ-210 is steam treated at 750°C for 1 hour in 100% steam followed by acid extraction.

4. A cobalt Fischer-Tropsch catalyst according to claim 1, in combination with an effective amount of a suitable promoter.

5. A cobalt Fischer-Tropsch catalyst according to claim 4 wherein the promoter is Mn oxide.

6. The use of a cobalt/steam treated, acid extracted LZ-210 molecular sieve supported catalyst according to claim 1 to catalyze a Fischer-Tropsch reaction.

7. The use of a cobalt steam treated acid extracted LZ-210 molecular sieve supported catalyst according to claim 6 wherein the LZ-210 molecular sieve support has a $SiO_2/Al_2O_3$ ratio of 8.0 or above.

8. The use of a cobalt steam treated acid extracted LZ-210 molecular sieve supported catalyst according to claim 6 wherein the LZ-210 molecular sieve support has been steam treated at 750°C for 1 hour in 100% steam followed by acid extraction.

9. The use of a cobalt steam treated, acid extracted LZ-210 molecular sieve supported catalyst according to claim 6, in combination with an effective amount of a suitable promoter, to catalyze a Fischer-Tropsch reaction.

10. The use of a cobalt steam treated, acid extracted LZ-210 molecular sieve supported catalyst according to claim 9, wherein the promoter is Mn oxide, to catalyze a Fischer-Tropsch reaction.

11. A process for carrying out a Fischer-Tropsch reaction on a syngas feed stream, comprising contacting said feed stream with a catalyst comprising cobalt and a steam treated, acid extracted LZ-210 molecular sieve support, at suitable Fischer-Tropsch reaction conditions.

12. A process for carrying out a Fischer-Tropsch reaction according to claim 11 wherein the LZ-210 molecular sieve support has a $SiO_2/Al_2O_3$ ratio of 8.0 or above.

13. A process for carrying out a Fischer-Tropsch reaction according to claim 11 wherein the LZ-210 molecular sieve support has been steam treated at 750°C for 1 hour in 100% steam followed by acid extraction.

14. A process for carrying out a Fischer-Tropsch reaction on a syngas feed stream, according to claim 11 wherein the catalyst also comprises an effective amount of a suitable promoter.

15. A process for carrying out a Fischer-Tropsch reaction, according to claim 14 wherein the catalyst also comprises a Mn oxide promoter.

16. A process for carrying out a Fischer-Tropsch reaction on a syngas feed stream, comprising contacting said feed stream with a catalyst comprising cobalt and a steam treated, acid extracted LZ-210 molecular sieve in combination with Mn oxide, at suitable Fischer-Tropsch reaction conditions.

| Category | Citation of document with indication, where appropriate, of relevant passages | Relevant to claim | CLASSIFICATION OF THE APPLICATION (Int. Cl.4) |
|---|---|---|---|
| A,D | US-A-4 652 538 (RABO et al.)<br>--- | | B 01 J 29/34<br>C 07 C 1/04 |
| A | EP-A-0 037 213 (MOBIL OIL CORP.)<br>--- | | |
| A | EP-A-0 153 517 (THE STANDARD OIL CO.)<br>----- | | |

**DOCUMENTS CONSIDERED TO BE RELEVANT**

European Patent Office

**EUROPEAN SEARCH REPORT**

Application Number

EP 88 11 4788

TECHNICAL FIELDS SEARCHED (Int. Cl.4)

C 07 C 1/00 ·
B 01 J 29/00

The present search report has been drawn up for all claims

| Place of search | Date of completion of the search | Examiner |
|---|---|---|
| THE HAGUE | 19-04-1989 | VAN GEYT J.J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)